# EUROPEAN PATENT APPLICATION

(11) **EP 4 527 335 A2**
(43) Date of publication of application: **26.03.2025**
(21) Application number: 24200966.0
(22) Date of filing: 18.09.2024
(51) Int. Cl.: A61B 34/20, A61B 34/10, A61B 90/00

(54) **3D VIRTUAL IMAGE WITH VIRTUAL LIGHTING TO TRACK MOVEMENT OF SENSOR-EQUIPPED MEDICAL INSTRUMENT**

(30) Priority: 19.09.2023 US 202363539118 P; 14.08.2024 US 202418804238
(71) Applicant: Biosense Webster (Israel) Ltd., 2066717 Yokneam (IL)
(72) Inventor: AUERBACH, Shmuel, 2066717 Yokneam (IL); NIGOVZIN, Igor, 2066717 Yokneam (IL); BELKIND-SHAFIR, Haya, 2066717 Yokneam (IL)
(74) Representative: Carpmaels & Ransford LLP

(57) **Abstract**

A position sensor of an instrument provides a signal indicating a real-time position and orientation of a distal portion of the instrument in response to an electromagnetic field. A processor drives a display screen to display an image of an anatomical region of a patient. The processor processes signals from the position sensor to determine the real-time position and orientation of a distal portion of the instrument within the patient, then provides a first indicator on the image to indicate the real-time position of the distal portion of the instrument within the patient. The processor also provides a second indicator on the image, indicating the real-time orientation of the distal portion of the instrument within the patient. The second indicator includes virtual illumination of an anatomical structure within the image. The virtually illuminated anatomical structure includes a structure toward which the distal portion of the instrument is oriented.

## Description

### PRIORITY

This application claims priority to U.S. Provisional Pat. App. No. 63/539,118, entitled "3D Virtual Image with Virtual Lighting to Track Movement of Sensor-Equipped Medical Instrument," filed September 19, 2023, the disclosure of which is incorporated by reference herein, in its entirety.

### BACKGROUND

Image-guided surgery (IGS) is a technique where a computer is used to obtain a real-time correlation of the location of an instrument that has been inserted into a patient's body to a set of preoperatively obtained images (e.g., a CT or MRI scan, 3-D map, etc.), such that the computer system may superimpose the current location of the instrument on the preoperatively obtained images. An example of an electromagnetic IGS navigation system that may be used in IGS procedures is the TRUDI^{®} Navigation System by Acclarent, Inc., of Irvine, California. In some IGS procedures, a digital tomographic scan (e.g., CT or MRI, 3-D map, etc.) of the operative field is obtained prior to surgery. A specially programmed computer is then used to convert the digital tomographic scan data into a digital map. During surgery, special instruments having sensors (e.g., electromagnetic coils that emit electromagnetic fields and/or are responsive to externally generated electromagnetic fields) are used to perform the procedure while the sensors send data to the computer indicating the current position of each surgical instrument. The computer correlates the data it receives from the sensors with the digital map that was created from the preoperative tomographic scan. The tomographic scan images are displayed on a video monitor along with an indicator (e.g., crosshairs or an illuminated dot, etc.) showing the real-time position of each surgical instrument relative to the anatomical structures shown in the scan images. The surgeon is thus able to know the precise position of each sensor-equipped instrument by viewing the video monitor even if the surgeon is unable to directly visualize the instrument itself at its current location within the body.

In some instances, it may be desirable to dilate an anatomical passageway in a patient. This may include dilation of ostia of paranasal sinuses (e.g., to treat sinusitis), dilation of the larynx, dilation of the Eustachian tube, dilation of other passageways within the ear, nose, or throat, etc. One method of dilating anatomical passageways includes using a guide wire and catheter to position an inflatable balloon within the anatomical passageway, then inflating the balloon with a fluid (e.g., saline) to dilate the anatomical passageway. For instance, the expandable balloon may be positioned within an ostium at a paranasal sinus and then be inflated, to thereby dilate the ostium by remodeling the bone adjacent to the ostium, without requiring incision of the mucosa or removal of any bone. The dilated ostium may then allow for improved drainage from and ventilation of the affected paranasal sinus.

It may also be desirable to ablate tissue within the ear, nose, or throat of a patient. For instance, such ablation may be desirable to remodel tissue (e.g., to reduce the size of a turbinate), to provide denervation (e.g., to disable the posterior nasal nerve), and/or for other purposes. Some such ablation treatments may include radiofrequency (RF) ablation with alternating current (AC) electrical energy; and/or irreversible electroporation (IRE) via pulsed field direct current (DC) electrical energy. To achieve ablation, an end effector with one or more needle electrodes or other kind(s) of tissue contacting electrodes may be activated with monopolar or bipolar electrical energy. Such ablation procedures may be carried out in conjunction with a dilation procedure or separately from a dilation procedure.

Some such procedures may be known as functional endoscopic sinus surgery (FESS). Such procedures may be performed using various instruments, such as microburs, shavers, drills, microdebriders, etc. Similarly, surgical cutting instruments may be used for removal of lesions, polyps and fibroids within the nasal cavity. In some such procedures, an endoscope may provide at least some degree of visualization of the surgical field. However, given the confines of the nasal cavity, it may be desirable to provide additional navigation guidance to the instrument via an IGS navigation system. Instruments such as those listed above may also be used in an otological context, such as during nerotology/lateral skull-based procedures. In such procedures, the options for providing endoscopic visualization may be even more limited than in the nasal cavity.

It may be desirable to provide enhanced real-time position and orientation information about one or more components of an ENT instrument. While several systems and methods have been made and used to position an ENT instrument in an anatomical passageway, it is believed that no one prior to the inventors has made or used the invention described in the appended claims.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of an example of a surgery navigation system being used on a patient seated in an example of a medical procedure chair;
FIG. 2 depicts a schematic view of an example of an instrument that may be used with the surgery navigation system of FIG. 1;
FIG. 3 depicts a flow chart representing an example of a process that may be carried out using the instrument of FIG. 2 and the surgery navigation system of FIG. 1;
FIG. 4A depicts an example of an image that may be rendered via a display screen of the surgery navigation system of FIG. 1 during performance of the process of FIG. 3 while the instrument of FIG. 2 is at a first position relative to a patient;
FIG. 4B depicts an example of an image that may be rendered via a display screen of the surgery navigation system of FIG. 1 during performance of the process of FIG. 3 while the instrument of FIG. 2 is at a second position relative to a patient;
FIG. 5 depicts an example of a set of images that may be rendered via a display screen of the surgery navigation system of FIG. 1 during performance of the process of FIG. 3;
FIG. 6 a depicts a flow chart representing an example of another process that may be carried out using the instrument of FIG. 2 and the surgery navigation system of FIG. 1;
FIG. 7A depicts an example of an image that may be rendered via a display screen of the surgery navigation system of FIG. 1 during performance of the process of FIG. 6 while the instrument of FIG. 2 is at a first position relative to a patient; and
FIG. 7B depicts an example of an image that may be rendered via a display screen of the surgery navigation system of FIG. 1 during performance of the process of FIG. 6 while the instrument of FIG. 2 is at a second position relative to a patient;

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

For clarity of disclosure, the terms "proximal" and "distal" are defined herein relative to a surgeon, or other operator, grasping a surgical instrument having a distal surgical end effector. The term "proximal" refers to the position of an element arranged closer to the surgeon, and the term "distal" refers to the position of an element arranged closer to the surgical end effector of the surgical instrument and further away from the surgeon. Moreover, to the extent that spatial terms such as "upper," "lower," "vertical," "horizontal," or the like are used herein with reference to the drawings, it will be appreciated that such terms are used for exemplary description purposes only and are not intended to be limiting or absolute. In that regard, it will be understood that surgical instruments such as those disclosed herein may be used in a variety of orientations and positions not limited to those shown and described herein.

As used herein, the terms "about" and "approximately" for any numerical values or ranges indicate a suitable dimensional tolerance that allows the part or collection of components to function for its intended purpose as described herein.

### I. Example of an Image Guided Surgery Navigation System

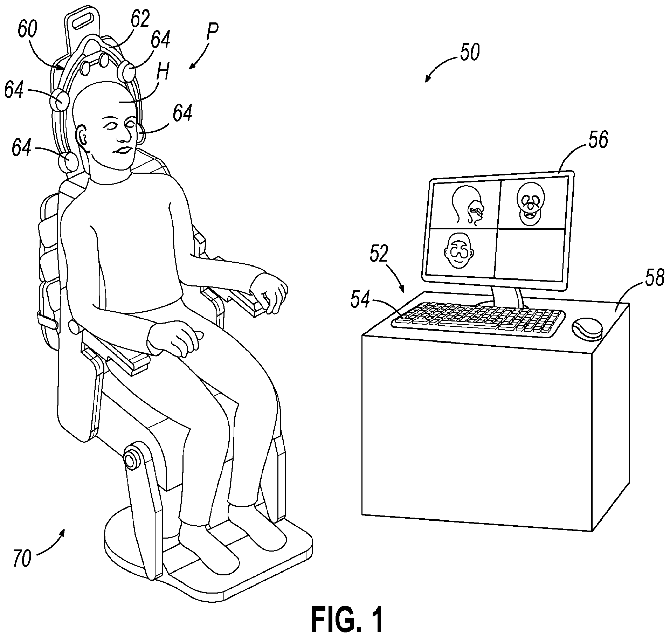

When performing a medical procedure within a head of a patient (P), it may be desirable to have information regarding the position of an instrument within the head (H) of the patient (P), particularly when the instrument is in a location where it is difficult or impossible to obtain an endoscopic view of a working element of the instrument within the head of the patient (P). FIG. 1 shows an example of an IGS navigation system (50) enabling a medical procedure to be performed within a head (H) of a patient (P) using image guidance. In addition to or in lieu of having the components and operability described herein IGS navigation system (50) may be constructed and operable in accordance with at least some of the teachings of U.S. Pat. No. 7,720,521, entitled "Methods and Devices for Performing Procedures within the Ear, Nose, Throat and Paranasal Sinuses," issued May 18, 2010, the disclosure of which is incorporated by reference herein, in its entirety; and/or U.S. Pat. No. 11,065,061, entitled "Systems and Methods for Performing Image Guided Procedures within the Ear, Nose, Throat and Paranasal Sinuses," issued July 20, 2021, the disclosure of which is incorporated by reference herein, in its entirety.

IGS navigation system (50) of the present example comprises a field generator assembly (60), which comprises a set of magnetic field generators (64) that are integrated into a horseshoe-shaped frame (62). Field generators (64) are operable to generate alternating magnetic fields of different frequencies around the head (H) of the patient (P). An instrument may be inserted into the head (H) of the patient (P). Such an instrument may include one or more position sensors as described in greater detail below. In the present example, frame (62) is mounted to a chair (70), with the patient (P) being seated in the chair (70) such that frame (62) is located adjacent to the head (H) of the patient (P). By way of example only, chair (70) and/or field generator assembly (60) may be configured and operable in accordance with at least some of the teachings of U.S. Pat. No. 10,561,370, entitled "Apparatus to Secure Field Generating Device to Chair," Issued February 18, 2020, the disclosure of which is incorporated by reference herein, in its entirety. In some other variations, the patient (P) lies on a table; and field generator assembly (60) is positioned on or near the table.

IGS navigation system (50) of the present example further comprises a processor (52), which controls field generators (64) and other elements of IGS navigation system (50). For instance, processor (52) is operable to drive field generators (64) to generate alternating electromagnetic fields; and process signals from the instrument to determine the location of a navigation sensor in the instrument within the head (H) of the patient (P). Processor (52) comprises a processing unit (e.g., a set of electronic circuits arranged to evaluate and execute software instructions using combinational logic circuitry or other similar circuitry) communicating with one or more memories. Processor (52) of the present example is mounted in a console (58), which comprises operating controls (54) that include a keypad and/or a pointing device such as a mouse or trackball. A physician uses operating controls (54) to interact with processor (52) while performing the surgical procedure.

While not shown, the instrument may include a navigation sensor that is responsive to positioning within the alternating magnetic fields generated by field generators (64). A coupling unit (not shown) may be secured to the proximal end of the instrument and may be configured to provide communication of data and other signals between console (58) and the instrument. The coupling unit may provide wired or wireless communication of data and other signals.

In some versions, the navigation sensor of the instrument may comprise at least one coil at or near the distal end of the instrument. When such a coil is positioned within an alternating electromagnetic field generated by field generators (64), the alternating magnetic field may generate electrical current in the coil, and this electrical current may be communicated as a signal along the electrical conduit(s) in the instrument and further to processor (52) via the coupling unit. This phenomenon may enable IGS navigation system (50) to determine the location of the distal end of the instrument within a three-dimensional space (i.e., within the head (H) of the patient (P), etc.). To accomplish this, processor (52) executes an algorithm to calculate location coordinates of the distal end of the instrument from the position related signals of the coil(s) in the instrument. Thus, a navigation sensor may serve as a position sensor by providing signals indicating the real-time position of the sensor within three-dimensional space.

Processor (52) uses software stored in a memory of processor (52) to calibrate and operate IGS navigation system (50). Such operation includes driving field generators (64), processing data from the instrument, processing data from operating controls (54), and driving display screen (56). In some implementations, operation may also include monitoring and enforcement of one or more safety features or functions of IGS navigation system (50). Processor (52) is further operable to provide video in real time via display screen (56), showing the position of the distal end of the instrument in relation to a video camera image of the patient's head (H), a CT scan image of the patient's head (H), and/or a computer-generated three-dimensional model of the anatomy within and adjacent to the patient's nasal cavity. Display screen (56) may display such images simultaneously and/or superimposed on each other during the surgical procedure. Such displayed images may also include graphical representations of instruments that are inserted in the patient's head (H), such that the operator may view the virtual rendering of the instrument at its actual location in real time. By way of example only, display screen (56) may provide images in accordance with at least some of the teachings of U.S. Pat. No. 10,463,242, entitled "Guidewire Navigation for Sinuplasty," issued November 5, 2019, the disclosure of which is incorporated by reference herein, in its entirety. In the event that the operator is also using an endoscope, the endoscopic image may also be provided on display screen (56).

The images provided through display screen (56) may help guide the operator in maneuvering and otherwise manipulating instruments within the patient's head (H). It should also be understood that other components of a surgical instrument and other kinds of surgical instruments, as described below, may incorporate a navigation sensor like the navigation sensor described above.

### II. Example of an ENT Instrument with a Position Sensor

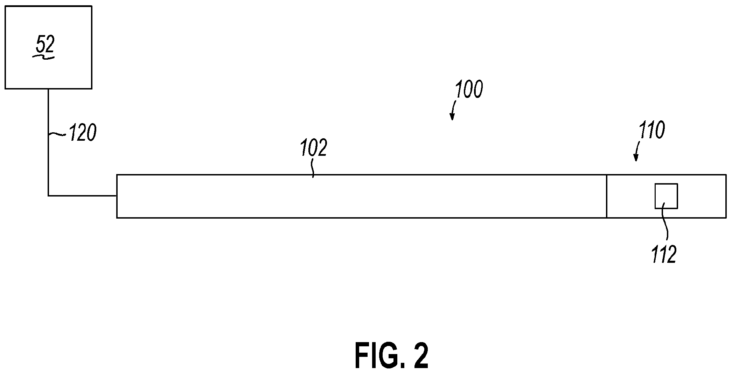

FIG. 2 shows an example of an instrument (100) that may be used with navigation system (50). Instrument (100) of this example includes a shaft (102) having an end effector (110) at the distal end of shaft (102). End effector (110) is configured to be inserted into the ear, nose, or throat of the patient (P) and perform some kind of operation within the patient (P). In some versions, end effector (110) includes an expandable dilator that is operable to dilate an anatomical passageway (e.g., paranasal sinus ostium, Eustachian tube, airway, etc.) within the ear, nose, or throat of the patient (P). In some other versions, end effector (110) includes one or more electrodes that is/are operable to provide ablative remodeling of tissue, denervation, or other effects. In still other versions, end effector (110) includes a rotary feature such as a microbur, shaver blade, drill bit, microdebrider, etc. End effector (110) may also include features to provide endoscopic visualization, illumination, irrigation, suction, and/or other effects. As yet another variation, end effector (110) may include one or more channels through which one or more other instruments may pass. Other suitable forms, features, and operabilities that may be provided in or through end effector (110) will be apparent to those skilled in the art in view of the teachings herein. Some variations of instrument (100) may also comprise a guidewire.

Instrument (100) further includes a position sensor (112) at end effector (110). Position sensor (112) is integrated in end effector (110) in this example, though other variations may provide position sensor (112) within a guidewire, guide rail, or other element that is movably disposed within end effector (110); or within a guide sheath or other element that is movably disposed about end effector (110). While only one position sensor (112) is shown, instrument (100) may include two or more position sensors (112), with position sensors (112) being located at any suitable position(s) along the length of shaft (102) and/or in end effector (110). In some versions where shaft (102) includes a steerable or otherwise deformable region, one or more position sensors (112) may be located distal to the steerable or otherwise deformable region while one or more other position sensors (112) may be located proximal to the steerable or otherwise deformable region, thereby facilitating determination of a bend angle formed by the steerable or otherwise deformable region. Position sensor (112) of the present example comprises one or more coils configured to provide position-indicative signals in response to alternating electromagnetic fields generated by field generators (64). The signals provided by position sensor (112) in response to such alternating electromagnetic fields may indicate the position of position sensor (112) in three-dimensional space. In addition, the signals provided by position sensor (112) in response to such alternating electromagnetic fields may indicate the orientation of position sensor (112) in three-dimensional space.

In the present example, instrument (100) is coupled with processor (52) via a cable (120) to communicate position-indicative signals from position sensor (112) to processor (52). In some other versions, instrument (100) is coupled with processor (52) wirelessly. In either case, processor (52) is operable to process the signals from position sensor (112) to thereby determine the real-time location and orientation of end effector (110) within a three-dimensional space (e.g., within the head (H) of the patient (P), etc.). Processor (52) is further operable to provide video in real time via display screen (56), showing the position of end effector (110) in relation to a video camera image of the patient's head (H), a CT scan image of the patient's head (H), and/or a computer-generated three-dimensional model of the anatomy within and adjacent to the patient's nasal cavity. Display screen (56) may display such images simultaneously and/or superimposed on each other during the surgical procedure. Such displayed images may also include a graphical representation of instrument (100), such that the operator may view the virtual rendering of instrument (100) at its actual location in real time. In some cases, the real-time position of end effector (110) is represented on display screen (56) by a crosshairs, dot, circle, or other form of visual indication.

### III. Example of User Interface Features for Use with IGS Navigation System

As noted above, processor (52) may drive display screen (56) to provide a visual indication of the real-time position of end effector (110) in relation to one or more images of the head (H) of the patient (P). In some cases, it may be beneficial for an operator to receive more real-time information via display screen (56), beyond just the real-time position of end effector (110) in three-dimensional space. Particularly when the operator is attempting to navigate end effector (110) along a tortuous pathway (e.g., within a nasal cavity), the operator may benefit from an enhanced view that provides an intuitive visual sense of the real-time orientation of end effector (110) within the patient (P). The operator may also benefit from an enhanced view that provides a real-time indication of proximity between end effector (110) and one or more identified anatomical regions. The following description provides examples of such enhancements that may be provided via display screen (56). While the examples are provided in the context of end effector (110) being used in an ear, nose, or throat procedure, the below teachings may be readily applied to other procedures within a head (H) of a patient (P) (e.g., intracranial navigation, such as for navigation toward a blood clot or aneurysm, etc.). Similarly, the below teachings may be readily applied in any other anatomical context, such that the following teachings are not limited to the context of procedures performed in the head (H) of a patient (P).

### A. Example of Real-time Position and Orientation Feedback via Virtual Illumination

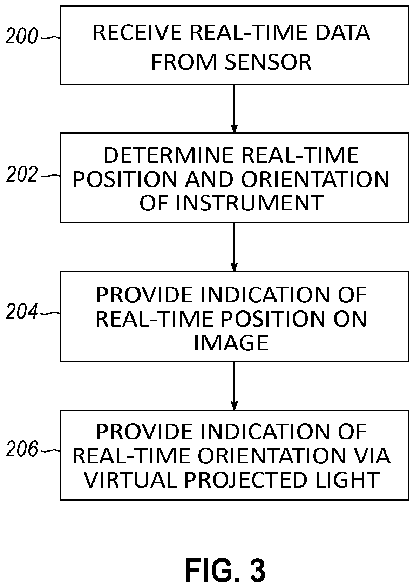

As noted above, it may be beneficial to provide an operator with an enhanced view via display screen (56) that provides an intuitive visual sense of the real-time orientation of end effector (110) within the patient (P). To that end, FIGS. 3-5 show an example of how processor (52) may drive display screen (56) to provide an intuitive visual indication of the position and orientation of end effector (110) via a virtual illumination projection. In particular, FIG. 3 shows an example of a process that begins with processor (52) receiving (block 200) real-time data from position sensor (112). As noted above, some variations of instrument (100) may include two or more position sensors (112), such that processor (52) may receive (block 200) real-time data from such two or more position sensors (112). With the real-time data from position sensor (112), processor (52) may then determine (block 202) the real-time position and orientation of instrument (100) in three-dimensional space. More specifically, processor (52) may determine (block 202) the real-time position and orientation of end effector (110) in the head (H) of the patient (P).

With the real-time position of end effector (110) having been determined, processor (52) may drive display screen (56) to provide (block 204) a visual indication of the real-time position of end effector (110) relative to one or more images of the patient (P). Similarly, with the real-time orientation of end effector (110) having been determined, processor (52) may drive display screen (56) to provide (block 206) a visual indication of the real-time orientation of end effector (110) relative to one or more images of the patient (P). This additional indication of the real-time orientation of end effector (110) may be more beneficial to the operator than just the indication of the real-time position of end effector (110), as the indication of the real-time orientation of end effector (110) may more clearly convey to the operator where end effector (110) will continue move as the operator further urges end effector (110) into the patient (P). Equipped with the knowledge of the real-time orientation of end effector (110), the operator may rotate end effector (110), steer end effector (110) (e.g., by articulating or bending shaft (102), etc.), or provide some other form of manipulation to re-orient end effector (110) as needed to continue urging end effector (110) down a desired path.

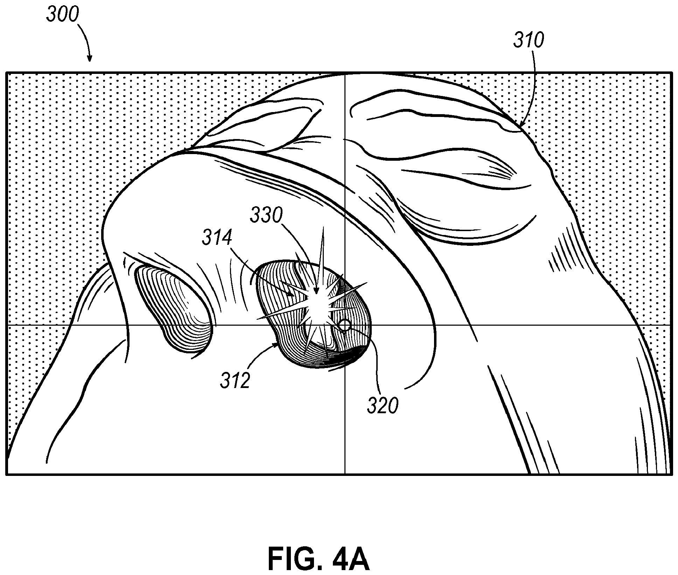

FIGS. 4A-4B show one example of how processor (52) may drive display screen (56) to provide (blocks 204, 206) a visual indication of the real-time position and orientation of end effector (110) relative to one or more images of the patient (P). In particular, FIG. 4A shows an example of an image (300) that may be rendered on display screen (56), where image (300) includes a three-dimensional virtual representation (310) of the head (H) of the patient (P). This image (300) includes a first indicator (320) representing the real-time position of end effector (110); and a second indicator (330) representing the real-time orientation of end effector (110). In the example shown, first indicator (320) is in the form of a crosshairs intersecting at the location corresponding to the real-time position of end effector (110). Alternatively, first indicator (320) may take any other suitable form, including but not limited to an "X," a circle, a dot, etc.

Second indicator (330) is in the form of a virtual illumination in the present example. In particular, second indicator (330) is shown as a light reflecting off the anatomical wall (as presented in virtual representation (310)) that would is intersected by a central longitudinal axis projecting distally from the distal end of end effector (110). In other words, second indicator (330) provides a visual appearance similar to what would be provided if a source of light were positioned at the distal end of end effector (110) (even though end effector (110) may lack a source of actual light), with the light from that simulated light source projecting directly toward (and reflecting off) the same anatomical structure toward which the distal end of end effector (110) is oriented. In the present example, the virtual illumination provided via second indicator (330) is similar to illumination that would be provided by a source of incoherent light, such that the virtual illumination is substantially concentrated within a central illuminated region with the illumination gently diffusing away from that central illuminated region. Image (300) may thus be analogous to an image that might be provided if instrument (100) were an endoscope, with end effector (110) including a camera and an incoherent light source, with the camera capturing the analog of image (300). To the extent that operators of instrument (100) have experience operating endoscopes and viewing the corresponding real-time endoscopic images, the format of image (300) and virtual illumination of second indicator (330) may provide such operators with an intuitive sense of the real-time orientation of end effector (110). Moreover, the virtual illumination of second indicator (330) may provide the operator with a better, more intuitive understanding of the contours and other surface features of anatomical structures (e.g., within a nasal cavity), particularly to the extent that the virtual illumination provides shadows and other effects that would be experience through actual illumination within anatomical cavities and passageways.

While second indicator (330) provides virtual illumination similar to illumination that would be provided by a source of incoherent light in the present example, the virtual illumination provided by second indicator (330) may take any other suitable form. By way of example only, the virtual illumination provided by second indicator (330) may be in the form of structured illumination providing a grid pattern or some other kind of pattern. As another example, the virtual illumination provided by second indicator (330) may be similar to illumination that would be provided by one or more sources of coherent light. Alternatively, the virtual illumination provided by second indicator (330) may take any other suitable form.

As shown in FIG. 4A, end effector (110) is positioned at the nostril of the patient (P) and is oriented toward the nasal cavity of the patient (P). Thus, first indicator (320) is positioned at a virtual representation (312) of the nostril of the patient (P); while second indicator (330) is positioned within a virtual representation (314) of the nasal cavity of the patient (P). With second indicator (330) is positioned within a virtual representation (314) of the nasal cavity of the patient (P), the operator will know that further advancement of end effector (110) will urge end effector (110) into the actual nasal cavity of the patient (P).

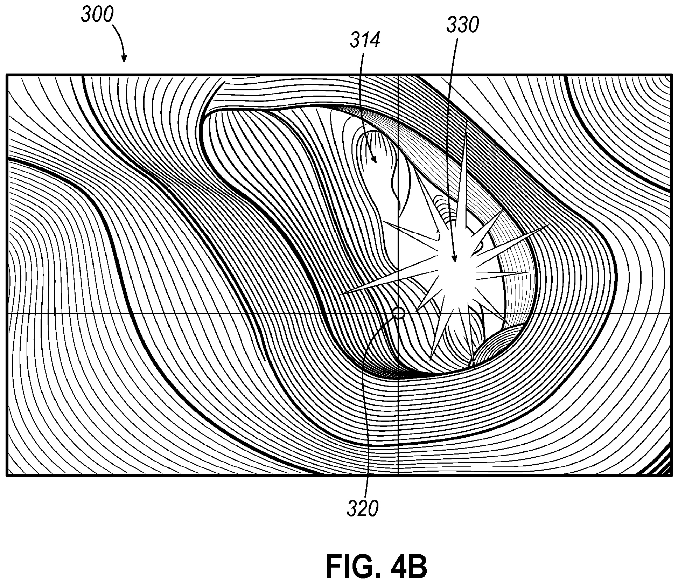

FIG. 4B shows image (300) after the operator has urged end effector (110) into the nasal cavity. As shown, first indicator (320) indicates the real-time location of end effector (110) within the virtual representation (314) of the nasal cavity of the patient (P); with second indicator (330) indicating that end effector (110) is oriented toward a region deeper into the nasal cavity of the patient (P).

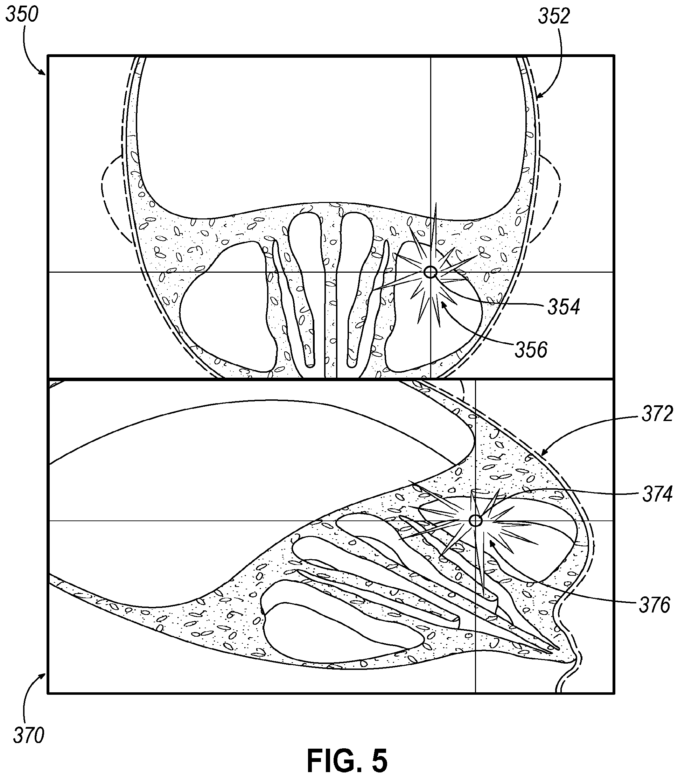

While FIGS. 4A-4B represent an analog to an endoscopic view from end effector (110) within a three-dimensional virtual representation (310) of the head (H) of the patient (P) (i.e., a virtually illuminated virtual endoscopic view), the real-time position and orientation of end effector (110) may be indicated in other ways via display screen (56). For instance, FIG. 5 shows another example of two images (350, 370) that may be rendered on display screen (56). Image (350) includes a top view of a cross-section (352) of the head (H) of the patient (P), taken along a horizontal plane. In some versions, image (350) comprises a preoperative image, such as CT scan image, MRI scan image, etc. A first indicator (354) in image (350) indicates the real-time position of end effector (110); and a second indicator (356) in image (350) indicates the real-time orientation of end effector (110). In the example shown, first indicator (354) is in the form of a crosshairs intersecting at the location corresponding to the real-time position of end effector (110). Alternatively, first indicator (354) may take any other suitable form, including but not limited to an "X," a circle, a dot, etc. Second indicator (356) is in the form of a virtual illumination. Thus, indicators (354, 356) in image (350) are configured and operable like indicators (320, 330) of image (300), respectively.

Image (370) includes a perspective, three-dimensional view of a virtual representation (372) of the head (H) of the patient (P), with a cross-section taken along the same horizontal plane as the cross-section in image (350). A first indicator (374) in image (370) indicates the real-time position of end effector (110); and a second indicator (376) in image (370) indicates the real-time orientation of end effector (110). In the example shown, first indicator (374) is in the form of a crosshairs intersecting at the location corresponding to the real-time position of end effector (110). Alternatively, first indicator (374) may take any other suitable form, including but not limited to an "X," a circle, a dot, etc. Second indicator (376) is in the form of a virtual illumination. Thus, indicators (374, 376) in image (370) are configured and operable like indicators (320, 330) of image (300), respectively.

While each image (350, 370) provides a cross-sectional view, with anatomical walls separating one cavity (e.g., maxillary sinus) from another cavity (e.g., meatus), there may be some instances where the virtual illumination from indicators (356, 376) may effectively pass through an anatomical wall from one cavity to another cavity, particularly when those cavities are connected to each other via a passageway that is not necessarily seen in the cross-sectional view of image (350, 370). For instance, in the views provided in images (350, 370) of FIG. 5, the virtual illumination of indicators (356, 376) originate from within a maxillary sinus cavity; and that virtual illumination may slightly "bleed" into the meatus region due to the presence of the maxillary sinus ostium providing a passageway through the lateral nasal wall between the maxillary sinus cavity and the meatus, even though the maxillary sinus ostium is inferior to the cross-sectional plane of images (350, 370). This "bleeding" of virtual light from indicators (356, 376) may vary with intensity as end effector (110) moves closer to the maxillary sinus ostium. The "bleeding" of virtual light from indicators (356, 376) may thus further indicate to the operator that end effector (110) is approaching a passageway (e.g., maxillary sinus ostium) connecting two cavities (e.g., the maxillary sinus cavity and the meatus) that are otherwise shown as being separate (e.g., separated by the lateral nasal wall). In other words, the "bleeding" of virtual light from indicators (356, 376) may further enhance the visual feedback to the operator of the real-time position and orientation of end effector (110), beyond the feedback that would be provided by indicators (356, 376) alone.

In the present example, both images (350, 370) are rendered on display screen (560 simultaneously; and are updated simultaneously in real time as end effector (110) moves within the patient (P). In some versions, both images (350, 370) and image (300) are all rendered on display screen (56) simultaneously; and are all updated simultaneously in real time as end effector (110) moves within the patient (P). Moreover, display screen (560) may further simultaneously render one or more other cross-sectional views (e.g., taken along a coronal plane and/or taken along a saggital plane), etc., in addition to or in lieu of providing images (300, 350, 370) described above. Such other cross-sectional views may be provided with indicators in a manner similar to that described above with respect to indicators (354, 356, 374, 376) in images (350, 370). Regardless of whether or how many cross-sectional views are provided via display screen (560), the cross-sectional plane of such views may move with end effector (110) in real time as end effector (110) moves within the head (H) of the patient (P), such that the position of the cross-sectional plane remains at the same depth as end effector (110) within the head (H) throughout movement of end effector (110) within the head (H).

### B. Example of Persistent Illumination of Identified Anatomical Regions

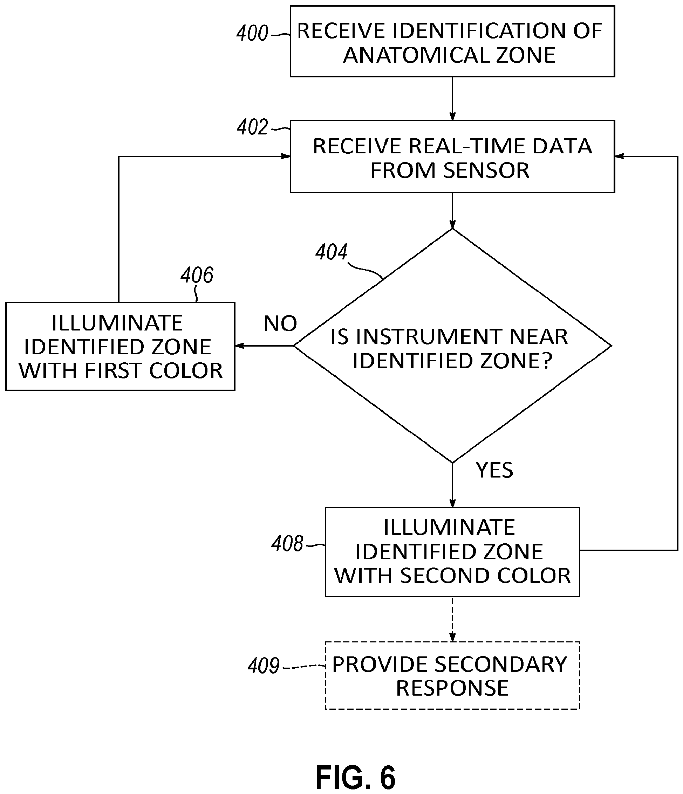

As noted above, it may be beneficial to provide an operator with an enhanced view via display screen (56) that provides a real-time indication of proximity between end effector (110) and one or more identified anatomical regions. In some cases, the one or more identified anatomical regions may represent intentional targets that the operator wishes to reach with end effector (110). In some other cases, the one or more identified anatomical regions may represent regions that the operator wishes to avoid with end effector (110) (e.g., due to a risk that end effector (110) may unintentionally damage an anatomical structure). FIGS. 6-7B show an example of how processor (52) may drive display screen (56) to provide a persistent indication of proximity between end effector (110) and one or more identified anatomical regions. In particular, FIG. 6 shows an example of a process that begins with processor (52) receiving (block 400) identification of an anatomical zone or structure. In some cases, the anatomical zone is identified by the operator. In some other cases, the anatomical zone is identified automatically. For instance, processor (52) may utilize artificial intelligence image processing techniques to identify one or more anatomical regions to intentionally target with end effector (110) and/or one or more anatomical regions to intentionally avoid with end effector (110). As another example, processor (52) may utilize artificial intelligence image processing techniques to suggest one or more anatomical regions to intentionally target with end effector (110) and/or one or more anatomical regions to intentionally avoid with end effector (110); and then the operator may manually select from those suggestions or otherwise modify the suggestions from processor (52).

Regardless of how the identified anatomical zone is identified, after processor (52) receives (block 400) the identified anatomical zone, processor (52) may receive (block 402) real-time data from position sensor (112) indicating the real-time position of end effector (110). Processor (52) may then compare the real-time position of end effector (110) against the position of the identified anatomical zone to determine (block 404) whether end effector (110) is within a certain proximity of the identified anatomical zone. The certain proximity may vary based on numerous factors, including but not limited to an operator's manual selection, the nature/sensitivity of the identified anatomical zone, the nature of end effector (110) (e.g., whether end effector (110) includes a rotary cutting member or an atraumatic tip, etc.), and/or other factors as will be apparent to those skilled in the art in view of the teachings herein.

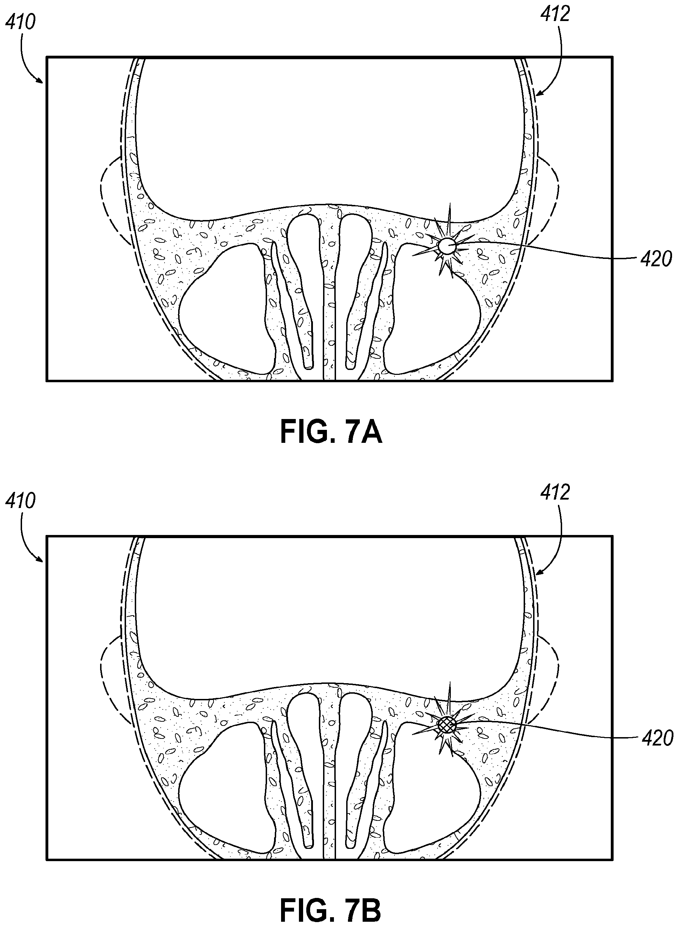

If processor (52) determines (block 404) that end effector (110) has not reached a certain proximity of the identified anatomical zone, then processor (52) drives display screen (56) to illuminate (block 406) an indicator (420) in an image (410) with a first color. FIG. 7A shows an example of how image (410) may appear. In this example, image (410) includes a top view of a cross-section (412) of the head (H) of the patient (P), taken along a horizontal plane. In some versions, image (410) comprises a preoperative image, such as CT scan image, MRI scan image, etc. Indicator (420) is in the form of a dot in this example; and is positioned at or near the location corresponding to the identified anatomical zone. In this example the identified anatomical zone is the ocular orbit, such that indicator (420) is positioned at the region of bone that is just inferior to the location of the ocular orbit. Also in this example, with the ocular orbit being the identified anatomical zone, this identified anatomical zone is identified as an anatomical zone that the operator wishes to avoid with end effector (110). As noted above, however, in some cases the anatomical zone may be an anatomical zone that the operator wishes to intentionally reach with end effector (110). Moreover, while only one anatomical zone is identified in this example, there may be other scenarios where two or more anatomical zones are identified; and such identified anatomical zones may be shown in image (410) via their own respective indicators (420).

By way of further example only, indicator (420) may be illuminated (block 406) with the color green in this scenario where processor (52) has determined (block 404) that end effector (110) has not reached a certain proximity of the identified anatomical zone. Alternatively, indicator (420) may be illuminated with any other suitable color; and/or may provide visual feedback in any other suitable form (e.g., different flashing frequency, different illumination intensity, etc.). Returning back to FIG. 6, while illuminating (block 406) indicator (420) in image (410) with the first color, processor (52) continues to receive (block 402) real-time data from position sensor (112) indicating the real-time position of end effector (110); and determining (block 404) whether end effector (110) is within a certain proximity of the identified anatomical zone. Processor (52) may thus persistently illuminate (block 406) indicator (420) in image (410) with the first color during the time where end effector (110) remains outside the certain proximity of the identified anatomical zone.

In the event that processor (52) determines (block 404) that end effector (110) has reached a certain proximity of the identified anatomical zone, then processor (52) drives display screen (56) to illuminate (block 408) indicator (420) in image (410) with a second color as shown in FIG. 7B. By way of example only, indicator (420) may be illuminated in green in FIG. 7A; and in red in FIG. 7B. The illumination (block 408) of indicator (420) in the second color (or second illumination pattern, etc.) may immediately visually indicate to the operator that end effector (110) has come within the certain proximity of the identified anatomical zone. In cases where the identified anatomical zone represents an anatomical structure that the operator wishes to avoid, the operator may cease advancement of end effector (110) and/or redirect the movement of end effector (110) to take end effector (110) out of the proximity of the identified anatomical structure in response to seeing indicator (420) illuminated (block 408) in the second color. In cases where the identified anatomical zone represents an anatomical structure that the operator wishes to intentionally reach with end effector (110), the operator may activate end effector (110) or perform some other operation in response to seeing indicator (420) illuminated (block 408) in the second color.

As with the illumination (block 406) associated with FIG. 7A, the illumination (block 408) associated with FIG. 7B may persist as processor (52) continues to receive (block 402) real-time data from position sensor (112) indicating the real-time position of end effector (110); and determining (block 404) that end effector (110) is within the certain proximity of the identified anatomical zone. Indicator (420) may transition from the second color back to the first color if and when processor (52) determines (block 404) that end effector (110) has moved out of the certain proximity of the identified anatomical zone.

In some cases, processor (52) may provide (block 409) one or more secondary responses in addition to illuminating (block 408) indicator in the second color in response to determining (block 404) that end effector (110) has come within the certain proximity of the identified anatomical zone. Such a secondary response may include an audible alert. In addition, or in the alternative, such a secondary response may include deactivation of end effector (110), or prevention of activation of end effector (110), until processor (52) determines (block 404) that end effector (110) has moved out of the certain proximity of the identified anatomical zone. Alternatively, secondary responses may take any other suitable form. It should be understood that providing (block 409) one or more secondary responses in addition to illuminating (block 408) indicator in the second color is merely optional, such that some versions may omit such one or more secondary responses.

While indicators (320, 330, 354, 356, 377, 376) are described above in a context that is separate from the context in which indicator (420) is described, it should be understood that the above teachings may be readily combined such that indicator (420) may be utilized in the same image (300, 350, 370) in which any of indicators (320, 330, 354, 356, 377, 376) appear. In other words, the teachings provided above with respect to indicators (320, 330, 354, 356, 377, 376) are not exclusive of the teachings provided above with respect to indicator (420).

### IV. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A system, comprising: (a) field generator assembly operable to generate an electromagnetic field around an anatomical region of a patient; (b) an instrument including a position sensor and a distal portion, the distal portion being sized for insertion into a patient, the position sensor being configured to provide a signal indicating a real-time position and orientation of the distal portion of the instrument within the patient in response to the electromagnetic field; (c) a display screen; and (d) a processor, the processor being configured to: (i) drive the display screen to display an image of the anatomical region of the patient, (ii) process signals from the position sensor to determine the real-time position and orientation of the distal portion of the instrument within the patient, (iii) provide a first indicator on the image, the first indicator indicating the real-time position of the distal portion of the instrument within the patient, and (iv) provide a second indicator on the image, the second indicator indicating the real-time orientation of the distal portion of the instrument within the patient, the second indicator including virtual illumination of an anatomical structure within the image, the virtually illuminated anatomical structure including a structure toward which the distal portion of the instrument is oriented.

### Example 2

The system of Example 1, the anatomical region of the patient including a head of the patient, the distal portion of the instrument being sized for insertion into the head of the patient.

### Example 3

The system of any of Examples 1 through 2, the position sensor comprising one or more coils.

### Example 4

The system of any of Examples 1 through 3, the distal portion of the instrument including an end effector, the end effector being operable to affect tissue.

### Example 5

The system of Example 4, the end effector comprising an expandable dilator.

### Example 6

The system of Example 4, the end effector comprising one or more electrodes.

### Example 7

The system of Example 4, the end effector including a rotary element selected from the group consisting of a microbur, shaver blade, drill bit, and microdebrider.

### Example 8

The system of Example 4, the end effector including an endoscopic visualization element.

### Example 9

The system of Example 4, the end effector including an illumination element.

### Example 10

The system of Example 4, the end effector including an irrigation opening configured to communicate irrigation fluid.

### Example 11

The system of Example 4, the end effector including a suction opening configured to communicate suction.

### Example 12

The system of Example 4, the end effector including a channel configured to slidably receive another instrument.

### Example 13

The system of any of Examples 1 through 12, the processor being further configured to: (i) drive the display screen to display an image of the anatomical region of the patient in the form of a three-dimensional virtual representation of the anatomical region of the patient, (ii) provide the first indicator on the three-dimensional virtual representation of the anatomical region of the patient, and (iii) provide the second indicator on the three-dimensional virtual representation of the anatomical region of the patient.

### Example 14

The system of Example 13, the processor being further configured to: (i) modify a viewing perspective of the three-dimensional virtual representation of the anatomical region of the patient in response to signals from the position sensor indicating repositioning of the distal portion of the instrument within the patient, (ii) provide the first indicator on the three-dimensional virtual representation of the anatomical region of the patient with the modified viewing perspective, and (iii) provide the second indicator on the three-dimensional virtual representation of the anatomical region of the patient with the modified viewing perspective.

### Example 15

The system of any of Examples 1 through 14, the processor being further configured to: (i) drive the display screen to display an image of the anatomical region of the patient in the form of a cross-sectional image of the anatomical region of the patient, (ii) provide the first indicator on the cross-sectional image of the anatomical region of the patient, and (iii) provide the second indicator on the cross-sectional image of the anatomical region of the patient.

### Example 16

The system of Example 15, the processor being further configured to: (i) modify a cross-sectional plane position of the cross-sectional image of the anatomical region of the patient in response to signals from the position sensor indicating repositioning of the distal portion of the instrument within the patient, (ii) provide the first indicator on the cross-sectional image of the anatomical region of the patient with the modified cross-sectional plane position, and (iii) provide the second indicator on the cross-sectional image of the anatomical region of the patient with the modified cross-sectional plane position.

### Example 17

The system of any of Examples 1 through 16, the processor being further configured to: (i) drive the display screen to simultaneously display two or more images of the anatomical region of the patient, (ii) provide the first indicator simultaneously on the two or more images the anatomical region of the patient, and (iii) provide the second indicator simultaneously on the two or more images the anatomical region of the patient.

### Example 18

The system of any of Examples 1 through 17, the virtual illumination providing a representation of illumination from incoherent light.

### Example 19

The system of any of Examples 1 through 18, the virtual illumination being configured to simulate illumination from a virtual light source positioned at a distal tip of the instrument.

### Example 20

The system of any of Examples 1 through 19, the distal portion of the instrument lacking a source of actual light.

### Example 21

The system of any of Example 1 through 20, the processor being further configured to: (i) persistently illuminate a third indicator on the image with a first illumination characteristic in response to determining that the distal portion of the instrument is outside a certain proximity of an identified anatomical structure, and (ii) persistently illuminate the third indicator on the image with a second illumination characteristic in response to determining that the distal portion of the instrument is within a certain proximity of the identified anatomical structure.

### Example 22

The system of Example 21, the first illumination characteristic comprising a first color, the second illumination characteristic comprising a second color.

### Example 23

The system of any of Examples 21 through 22, the first illumination characteristic comprising a first flash frequency, the second illumination characteristic comprising a second flash frequency.

### Example 24

The system of any of Examples 21 through 23, the first illumination characteristic comprising a first illumination intensity, the second illumination characteristic comprising a second illumination intensity.

### Example 25

A method comprising: (a) driving a display screen to display an image of an anatomical region of a patient; (b) processing signals from a position sensor of an instrument to determine the real-time position and orientation of a distal portion of the instrument within the patient; (c) providing a first indicator on the image, the first indicator indicating the real-time position of the distal portion of the instrument within the patient; and (d) providing a second indicator on the image, the second indicator indicating the real-time orientation of the distal portion of the instrument within the patient, the second indicator including virtual illumination of an anatomical structure within the image, the virtually illuminated anatomical structure including a structure toward which the distal portion of the instrument is oriented.

### Example 26

The method of Example 25, the anatomical region of the patient including a head of the patient, the distal portion of the instrument being inserted in the head of the patient.

### Example 27

The method of any of Examples 25 through 26, the displayed image comprising a three-dimensional virtual representation of the anatomical region of the patient, the first indicator being provided on the three-dimensional virtual representation of the anatomical region of the patient, the second indicator being provided on the three-dimensional virtual representation of the anatomical region of the patient.

### Example 28

The method of Example 27, further comprising: (a) modifying a viewing perspective of the three-dimensional virtual representation of the anatomical region of the patient in response to signals from the position sensor indicating repositioning of the distal portion of the instrument within the patient; (b) providing the first indicator on the three-dimensional virtual representation of the anatomical region of the patient with the modified viewing perspective; and (c) providing the second indicator on the three-dimensional virtual representation of the anatomical region of the patient with the modified viewing perspective.

### Example 29

The method of any of Examples 25 through 28, the displayed image comprising a cross-sectional image of the anatomical region of the patient, the first indicator being provided on the cross-sectional image of the anatomical region of the patient, the second indicator being provided on the cross-sectional image of the anatomical region of the patient.

### Example 30

The method of Example 29, further comprising: (a) modifying a cross-sectional plane position of the cross-sectional image of the anatomical region of the patient in response to signals from the position sensor indicating repositioning of the distal portion of the instrument within the patient; (b) providing the first indicator on the cross-sectional image of the anatomical region of the patient with the modified cross-sectional plane position; and (c) providing the second indicator on the cross-sectional image of the anatomical region of the patient with the modified cross-sectional plane position.

### Example 31

The method of any of Examples 25 through 30, further comprising: (a) driving the display screen to simultaneously display two or more images of the anatomical region of the patient; (b) providing the first indicator simultaneously on the two or more images the anatomical region of the patient; and (c) providing the second indicator simultaneously on the two or more images the anatomical region of the patient.

### Example 32

The method of any of Examples 25 through 31, the virtual illumination providing a representation of illumination from incoherent light.

### Example 33

The method of any of Examples 25 through 32, the virtual illumination simulating illumination from a virtual light source positioned at a distal tip of the instrument.

### Example 34

The method of any of Examples 25 through 33, the distal portion of the instrument lacking a source of actual light.

### Example 35

The method of any of Examples 25 through 33, further comprising: (a) persistently illuminating a third indicator on the image with a first illumination characteristic in response to determining that the distal portion of the instrument is outside a certain proximity of an identified anatomical structure; and (b) persistently illuminating the third indicator on the image with a second illumination characteristic in response to determining that the distal portion of the instrument is within a certain proximity of an identified anatomical structure.

### Example 36

The method of Example 35, the first illumination characteristic comprising a first color, the second illumination characteristic comprising a second color.

### Example 37

The method of any of Examples 35 through 36, the first illumination characteristic comprising a first flash frequency, the second illumination characteristic comprising a second flash frequency.

### Example 38

The method of any of Examples 35 through 37, the first illumination characteristic comprising a first illumination intensity, the second illumination characteristic comprising a second illumination intensity.

### Example 39

A system, comprising: (a) field generator assembly operable to generate an electromagnetic field around an anatomical region of a patient; (b) an instrument including a position sensor and a distal portion, the distal portion being sized for insertion into a patient, the position sensor being configured to provide a signal indicating a real-time position and orientation of the distal portion of the instrument within the patient in response to the electromagnetic field; (c) a display screen; and (d) a processor, the processor being configured to: (i) drive the display screen to display an image of the anatomical region of the patient, (ii) process signals from the position sensor to determine the real-time position of the distal portion of the instrument within the patient, (iii) persistently illuminate a first indicator on the image with a first illumination characteristic in response to determining that the distal portion of the instrument is outside a certain proximity of an identified anatomical structure, and (iv) persistently illuminate the first indicator on the image with a second illumination characteristic in response to determining that the distal portion of the instrument is within a certain proximity of the identified anatomical structure.

### Example 40

The system of Example 39, the first illumination characteristic comprising a first color, the second illumination characteristic comprising a second color.

### Example 41

The system of any of Examples 39 through 40, the first illumination characteristic comprising a first flash frequency, the second illumination characteristic comprising a second flash frequency.

### Example 42

The system of any of Examples 39 through 41, the first illumination characteristic comprising a first illumination intensity, the second illumination characteristic comprising a second illumination intensity.

### Example 43

The system of any of Examples 39 through 42, the first indicator being positioned within a region of the image corresponding to the identified anatomical structure.

### Example 44

The system of any of Examples 39 through 43, the processor being further configured to provide a second indicator on the image, the second indicator indicating the real-time position of the distal portion of the instrument within the patient.

### Example 45

The system of any of Examples 39 through 44, the processor being further configured to: (i) process signals from the position sensor to determine the real-time orientation of the distal portion of the instrument within the patient, and (ii) provide a third indicator on the image, the third indicator indicating the real-time orientation of the distal portion of the instrument within the patient, the third indicator including virtual illumination of an anatomical structure within the image, the virtually illuminated anatomical structure including a structure toward which the distal portion of the instrument is oriented.

### Example 46

A method comprising: (a) driving a display screen to display an image of an anatomical region of a patient; (b) processing signals from a position sensor to determine the real-time position of a distal portion of an instrument within the patient; (c) determining whether the distal portion of the instrument is within a certain proximity of an identified anatomical structure; and (d) persistently illuminating a first indicator on the image with a first illumination characteristic while the distal portion of the instrument is outside the certain proximity of the identified anatomical structure; and (e) if the distal portion of the instrument reaches the certain proximity of the identified anatomical structure, persistently illuminating the first indicator on the image with a second illumination characteristic to thereby indicate that the distal portion of the instrument is within the certain proximity of the identified anatomical structure.

### Example 47

The method of Example 46, further comprising receiving operator input indicating the identified anatomical structure.

### Example 48

The method of any of Examples 46 through 47, further comprising automatically identifying the identified anatomical structure.

### Example 49

The method of any of Examples 46 through 48, the first illumination characteristic comprising a first color, the second illumination characteristic comprising a second color.

### Example 50

The method of any of Examples 46 through 48, the first illumination characteristic comprising a first flash frequency, the second illumination characteristic comprising a second flash frequency.

### Example 51

The method of any of Examples 46 through 50, the first illumination characteristic comprising a first illumination intensity, the second illumination characteristic comprising a second illumination intensity.

### Example 52

The method of any of Examples 46 through 51, the first indicator being positioned within a region of the image corresponding to the identified anatomical structure.

### Example 53

The method of any of Examples 46 through 52, the anatomical region of the patient including a head of the patient, the distal portion of the instrument being inserted in the head of the patient.

### Example 54

The method of any of Examples 46 through 53, further comprising providing a second indicator on the image, the second indicator indicating the real-time position of the distal portion of the instrument within the patient.

### Example 55

The system of any of Examples 46 through 54, further comprising: (a) processing signals from the position sensor to determine the real-time orientation of the distal portion of the instrument within the patient; and (b) providing a third indicator on the image, the third indicator indicating the real-time orientation of the distal portion of the instrument within the patient, the third indicator including virtual illumination of an anatomical structure within the image, the virtually illuminated anatomical structure including a structure toward which the distal portion of the instrument is oriented.

### V. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A system, comprising:
(a) field generator assembly operable to generate an electromagnetic field around an anatomical region of a patient;
(b) an instrument including a position sensor and a distal portion, the distal portion being sized for insertion into a patient, the position sensor being configured to provide a signal indicating a real-time position and orientation of the distal portion of the instrument within the patient in response to the electromagnetic field;
(c) a display screen; and
(d) a processor, the processor being configured to:
(i) drive the display screen to display an image of the anatomical region of the patient,
(ii) process signals from the position sensor to determine the real-time position and orientation of the distal portion of the instrument within the patient,
(iii) provide a first indicator on the image, the first indicator indicating the real-time position of the distal portion of the instrument within the patient, and
(iv) provide a second indicator on the image, the second indicator indicating the real-time orientation of the distal portion of the instrument within the patient, the second indicator including virtual illumination of an anatomical structure within the image, the virtually illuminated anatomical structure including a structure toward which the distal portion of the instrument is oriented.

2. The system of claim 1, the anatomical region of the patient including a head of the patient, the distal portion of the instrument being sized for insertion into the head of the patient.

3. The system of claim 1 or claim 2, the position sensor comprising one or more coils.

4. The system of any preceding claim, the distal portion of the instrument including an end effector, the end effector being operable to affect tissue.

5. The system of claim 4, the end effector comprising one or more features selected from the group consisting of: an expandable dilator, one or more electrodes, a microbur, a shaver blade, a drill bit, a microdebrider, an illumination element, an irrigation opening, a suction opening, and a channel configured to slidably receive another instrument.

6. The system of any preceding claim, the processor being further configured to:
(i) drive the display screen to display an image of the anatomical region of the patient in the form of a three-dimensional virtual representation of the anatomical region of the patient,
(ii) provide the first indicator on the three-dimensional virtual representation of the anatomical region of the patient, and
(iii) provide the second indicator on the three-dimensional virtual representation of the anatomical region of the patient.

7. The system of claim 6, the processor being further configured to:
(i) modify a viewing perspective of the three-dimensional virtual representation of the anatomical region of the patient in response to signals from the position sensor indicating repositioning of the distal portion of the instrument within the patient,
(ii) provide the first indicator on the three-dimensional virtual representation of the anatomical region of the patient with the modified viewing perspective, and
(iii) provide the second indicator on the three-dimensional virtual representation of the anatomical region of the patient with the modified viewing perspective.

8. The system of any preceding claim, the processor being further configured to:
(i) drive the display screen to display an image of the anatomical region of the patient in the form of a cross-sectional image of the anatomical region of the patient,
(ii) provide the first indicator on the cross-sectional image of the anatomical region of the patient, and
(iii) provide the second indicator on the cross-sectional image of the anatomical region of the patient.

9. The system of any preceding claim, the processor being further configured to:
(i) drive the display screen to simultaneously display two or more images of the anatomical region of the patient,
(ii) provide the first indicator simultaneously on the two or more images the anatomical region of the patient, and
(iii) provide the second indicator simultaneously on the two or more images the anatomical region of the patient.

10. The system of any preceding claim, the virtual illumination providing a representation of illumination from incoherent light.

11. The system of any preceding claim, the virtual illumination being configured to simulate illumination from a virtual light source positioned at a distal tip of the instrument.

12. The system of any preceding claim, the distal portion of the instrument lacking a source of actual light.

13. The system of any preceding claim, the processor being further configured to:
(i) persistently illuminate a third indicator on the image with a first illumination characteristic in response to determining that the distal portion of the instrument is outside a certain proximity of an identified anatomical structure, and
(ii) persistently illuminate the third indicator on the image with a second illumination characteristic in response to determining that the distal portion of the instrument is within a certain proximity of the identified anatomical structure.

14. The system of claim 13, the first illumination characteristic comprising a first color, the second illumination characteristic comprising a second color, and/or the first illumination characteristic comprising a first flash frequency, the second illumination characteristic comprising a second flash frequency, and/or the first illumination characteristic comprising a first illumination intensity, the second illumination characteristic comprising a second illumination intensity.

15. A system, comprising:
(a) field generator assembly operable to generate an electromagnetic field around an anatomical region of a patient;
(b) an instrument including a position sensor and a distal portion, the distal portion being sized for insertion into a patient, the position sensor being configured to provide a signal indicating a real-time position and orientation of the distal portion of the instrument within the patient in response to the electromagnetic field;
(c) a display screen; and
(d) a processor, the processor being configured to:
(i) drive the display screen to display an image of the anatomical region of the patient,
(ii) process signals from the position sensor to determine the real-time position of the distal portion of the instrument within the patient,
(iii) persistently illuminate a first indicator on the image with a first illumination characteristic in response to determining that the distal portion of the instrument is outside a certain proximity of an identified anatomical structure, and
(iv) persistently illuminate the first indicator on the image with a second illumination characteristic in response to determining that the distal portion of the instrument is within a certain proximity of the identified anatomical structure.
